**Europäisches Patentamt**

**European Patent Office**

**Office européen des brevets**

(19)

(11) **EP 1 024 786 B1**

(12) **EUROPÄISCHE PATENTSCHRIFT**

(45) Veröffentlichungstag und Bekanntmachung des Hinweises auf die Patenterteilung:
**18.12.2002 Patentblatt 2002/51**

(51) Int Cl.$^7$: **A61K 7/50**

(21) Anmeldenummer: **98958227.5**

(86) Internationale Anmeldenummer:
**PCT/EP98/06680**

(22) Anmeldetag: **21.10.1998**

(87) Internationale Veröffentlichungsnummer:
**WO 99/022712 (14.05.1999 Gazette 1999/19)**

(54) **HAUTVERTRÄGLICHE HANDREINIGER, INSBESONDERE GROBHANDREINIGER**

SKIN COMPATIBLE HAND CLEANSER, ESPECIALLY A COURSE HAND CLEANSER

DETERGENTS A TOLERANCE CUTANEE DESTINES AUX MAINS, NOTAMMENT DETERGENTS DESTINES AU NETTOYAGE GROSSIER DES MAINS.

(84) Benannte Vertragsstaaten:
**AT BE CH CY DE DK ES FI FR GB GR IE IT LI LU MC NL PT SE**

(30) Priorität: **30.10.1997 DE 19748921**

(43) Veröffentlichungstag der Anmeldung:
**09.08.2000 Patentblatt 2000/32**

(73) Patentinhaber: **Stockhausen GmbH**
**47805 Krefeld (DE)**

(72) Erfinder:
• **ROSENBERGER, Volker**
**D-41564 Kaarst (DE)**
• **KLOTZ, Andreas**
**D-47798 Krefeld (DE)**
• **VEEGER, Marcel**
**D-47807 Krefeld (DE)**
• **BRÜCHER, Beatrice**
**D-47807 Krefeld (DE)**

(74) Vertreter: **Kutzenberger, Helga, Dr.**
**Kutzenberger & Wolff,**
**Theodor-Heuss-Ring 23**
**50668 Köln (DE)**

(56) Entgegenhaltungen:
**EP-A- 0 557 825**      **EP-A- 0 769 292**
**DE-A- 4 424 210**

**Beschreibung**

Die Erfindung betrifft wasserhaltige flüssige, pasten- oder cremeförmige Handreinigungsmittel mit verbesserter dermatologischer Verträglichkeit und die Verwendung der Mittel zur Entfernung von extremen Verschmutzungen.

Grobhandreiniger werden vorzugsweise dort angewendet, wo mit Verschmutzungen, wie Lacke, Fette, Öle, Schmierstoffe, Metallstäube, Graphit, Ruß u. ä., zu rechnen ist. Derartige Reinigungsmittel sind beispielsweise nach H. Tronnier, J. Kresken, K. Jablonski, B. Komp, Haut und Beruf, Grosse Verlag, Berlin, 1989, S. 75 -108 bekannt. Im allgemeinen kennt man solche Zubereitungen, die ein Abrasivum, Tensid/Tensidgemische, Verdickungsmittel sowie gegebenenfalls Hilfsstoffe zur Regulierung der Konsistenz, von Aussehen, Geruch und Stabilität, wie Pigmente, Duftstoffe, Stabilisatoren und Konservierungsmittel enthalten. Für besonders hartnäckige Verschmutzungen stehen Produkte zur Verfügung, bei denen organische Lösungsmittel zugesetzt werden, wie z. B. aliphatische Kohlenwasserstoffe, Terpene, Carbonsäureester vom Typ Dimethyladipat, Dimethylglutarat, Dimethlylsuccinat (DBE) und Di-n-butyladipat bzw. Di-isopropyladipat, wie sie in der DE 43 35 933 A1 beschrieben worden sind.

Je häufiger derartige Produkte im industriellen Bereich (bis zu 6mal und öfter am Tag) auf der Haut angewendet werden, desto deutlicher treten die nachteiligen Wirkungen der Tenside und insbesondere der Lösungsmittel, nämlich die Entfettung und Austrocknung der Haut durch die Zerstörung des Hydro-Lipid-Mantels der Haut, in den Vordergrund. Es kommt dann zu verstärkter Resorption toxischer und allergener Stoffe oder zum Befall von Mikroorganismen oder als Folge zu toxischen oder allergenen Hautreaktionen.

In jüngster Zeit sind Grobhandreiniger bekannt, die neben den Tensiden Cocosnußfettsäuremonoethanolamid, Cocamidopropyldimethylglycin, C9-11-Pareth-6 und sulfatiertem Rizinusöl ein Traubenkernöl enthalten. Diese Mittel sind hinsichtlich ihrer Unterschiedlichkeit und ihrer Reinigungskraft eingeschränkt. Weiterhin ist ein Sensibilisierungsrisiko bei den Tensiden Cocosnußfettsäuremonoethanolamid und Cocamidopropylbetain bekannt.

Die DE 44 24 210 A1 beschreibt wasserfreie Duschöle, die mindestens 45 Gew.% Öl, ausgewählt aus der Gruppe der Öle mit hohem Gehalt an Triglyceriden, gesättigten und/oder ungesättigten Fettsäuren, wie z. B. Sojaöl, Sonnenblumenöl, Weizenkeimöl, und höchstens 55 Gew.% Tenside, vorzugsweise Monoisopropanolaminlaurylethersulfat, Cocosfettsäurediethanolamid, enthalten. Diese Zubereitungen haben folgende Nachteile: 1. reicht ihre Reinigungskraft nicht aus, um extreme Verschmutzungen zu entfernen, und 2. enthalten sie Fettsäuremono- bzw. diethanolamide, von denen nach Fiedler, Edito Cantor Verlag, 4. Aufl., 1996, S. 376 ein Sensibilisierungsrisiko bekannt ist.

Aus der EP 0 769 292 A1 sind wasserhaltige Duschzubereitungen bekannt, wobei neben Tensidgemischen Rüböl oder Rüböl-Derivate als Hautpflegekomponenten verwendet werden. Auch diese Mittel sind wegen der fehlenden Reinigungskraft als Grobhandreiniger nicht geeignet.

Wegen der beschriebenen nachteiligen Wirkungen der herkömmlichen Mittel besteht daher die Aufgabe, ein aus dermatologischer Sicht sehr gut hautverträgliches Handreinigungsmittel, vorzugsweise ein Grobhandreinigungsmittel bereitzustellen, das auch bei mehrmaliger täglicher Anwendung auf der gesunden Haut nur eine geringe Hautaustrocknung aufweist. Weiterhin sollte auf Tenside verzichtet werden, die ein Sensibilisierungsrisiko aufweisen.

Erstaunlich und anhand des Standes der Technik nicht vorauszusagen war, daß wasserhaltige, lösungsmittelfreie, flüssige, pasten- oder cremeförmige Handreiniger, insbesondere Grobhandreiniger erhältlich sind, welche die gestellte Aufgabe erfüllen.

Diese sind gekennzeichnet durch einen Gehalt von

a) 10 - 30 Gew.% eines oder mehrerer pflanzlicher Öle, bezogen auf die Zusammensetzung des Handreinigungsmittels, aus der Gruppe der Triglyceride gesättigter und/oder ungesättigter Fettsäuren, bevorzugt Triglyceride mit einem höheren Anteil an ungesättigten Fettsäuren, besonders bevorzugt Rüböl, Sojaöl und/oder Leinöl
b) 10 - 30 Gew.%, bezogen auf die Zusammensetzung des Handreinigungsmittels, einer Tensid-Komposition, die

α) wenigstens ein Fettalkoholethoxylat,
β) wenigstens ein Fettalkoholethersulfat und
γ) wenigstens ein Salz einer sulfierten, vorzugsweise sulfonierten Fettsäure enthält

c) 10 - 65 Gew.%, bezogen auf die Zusammensetzung des Handreinigungsmittels, Wasser
d) gegebenenfalls 1 - 30 Gew.%, bezogen auf die Zusammensetzung des Handreinigungsmittels, eines oder mehrerer Abrasiva, bevorzugt Kunststoffreibernittel auf der Basis von Polyethylen oder Polyurethan, Reibemittel auf der Basis von natürlichen Kern- und/oder Schalenmehlen, bevorzugt gebleichten natürlichen Schalen- und/oder Kernmehlen wie beispielsweise $H_2O_2$ gebleichtes Walnußschalen-, Mandelschalen- oder Haselnußschalenmehl, Oliven-, Aprikosen- oder Kirschkernmehl oder beliebige Gemische dieser Schalen- und Kernmehle, besonders bevorzugt $H_2O_2$ gebleichtes Walnußschalenmehl, und/oder Perlen aus Wachsen, bevorzugt Jojobawachsen,
e) gegebenenfalls ein oder mehrere viskositätsbildende Mittel, bevorzugt organophile und/oder hydrophile Schichtsilikate, besonders bevorzugt Bentonite, Polysaccharide, bevorzugt Cellulose, Guarmehl und/oder Xanthane, modifizierte Polysaccharide, vorzugsweise Celluloseether, Carboxymethylcellulose und/oder Hydroxyethylcellulose und/oder anorganische Elektrolyte, vorzugsweise NaCl und/oder $MgSO_4$,

f) gegebenenfalls weitere kosmetische Hilfs-, Zusatz- und/oder Wirkstoffe, wie pH-Regulatoren, Duftstoffe, Konservierungsmittel, wie organische Säuren, und Antioxidantien, wie Vitamin E-Acetat,

wobei die Summe der Komponenten a) bis f) immer 100 Gew.% ergeben muß und wobei der Gehalt der Komponente a) größer oder gleich dem Gehalt an Fettalkoholethoxylaten α) und der Gehalt an Fettalkoholethoxylaten a) größer oder gleich dem Gehalt der beiden Komponenten β) und γ) in der Zusammensetzung des Handreinigungsmittels ist.

Vorzugsweise werden in der Komponente b) Fettalkoholethoxylate der Struktur

$$R\text{-}O\text{-}(CH_2\text{-}CH_2\text{-}O)_n H$$

mit

R = einem $C_8$-$C_{18}$, vorzugsweise $C_{10}$-$C_{16}$, besonders bevorzugt $C_{11}$-$C_{14}$ gesättigten oder ungesättigten, verzweigten oder unverzweigten Alkylrest, und n = einer ganzen Zahl von 1 - 8, vorzugsweise 3 - 6, besonders bevorzugt 5 - 7, eingesetzt und insbesondere wird bevorzugt Laureth-6 mit R = $C_{12}$ und n = 6 verwendet.

Vorzugsweise zu verwendende Fettalkoholethersulfate in Komponente b) sind solche der allgemeinen Formel

$$R\text{-}O\text{-}(CH_2CH_2\text{-}O)_n\, SO_3\, X$$

mit R = einem $C_{10}$-$C_{16}$, bevorzugt $C_{11}$-$C_{14}$ gesättigten oder ungesättigten, verzweigten oder unverzweigten Alkylrest, n = einer ganzen Zahl von 1 - 6, vorzugsweise 1 - 4, und X = $Na^-$, $NH_4^+$ oder $Mg^{2+}$, besonders bevorzugt wird Natriumlaurylethersulfat (mit R = $C_{12}$, n = 2 - 3 und X = $Na^+$) eingesetzt.

Als Salze von sulfierten, vorzugsweise sulfonierten Fettsäuren werden Alkali- oder Erdalkalisalze von $C_8$-$C_{30}$, bevorzugt $C_{10}$-$C_{22}$ Fettsäuren, besonders bevorzugt Rizinusölsulfate, insbesondere $Na^+$ oder $NH_4^+$ Sulfate verwendet.

Als Rizinusölsulfonat ist Monobrilliantöl® (Stockhausen GmbH & Co. KG) oder Standapol SCO® (Henkel KGaA) geeignet,

Die erfindungsgemäße zum Einsatz kommende Tensidkompositionen weisen keine (oder nur eine äußerst geringe) sensibilisierende Wirkung auf.

Es wurde überraschenderweise gefunden, daß die vorliegende Aufgabe nur dann erfüllt werden kann, wenn die Bedingung eingehalten wird, daß in der Zusammensetzung der Grobhandreiniger der Gehalt an pflanzlichem Öl größer oder gleich dem Gehalt an Fettalkoholethoxylaten ist und der Gehalt an Fettalkoholethoxylaten größer oder gleich dem Gehalt der beiden Komponenten Fettalkoholethersulfat und sulfierten Fettsäuren ist.

Dementsprechend ist der Gehalt von 10 - 30 Gew.% Tensidkomposition auf 5 - 28 Gew.%, vorzugsweise 7 - 25 Gew.%, der Komponente α,
1,9 - 15 Gew.%, vorzugsweise 3 - 10 Gew.% der Komponente β und
0,1 - 10 Gew.%, vorzugsweise 0,5 - 6 Gew.% der Komponente γ aufgeteilt.

Ganz besonders bevorzugt besteht der Anteil von 10 - 30 Gew.% der Tensidkomposition aus 5 - 28 Gew.% Laureth-6,
1 - 10 Gew.% Natriumlaurethsulfat (= Natriumlaurylethersulfat mit 2 bis 3 EO) und 1 - 10 Gew.% Rizinusölsulfonat.

Die erfindungsgemäßen Handreinigungsmittel enthalten keine organischen Lösungsmittel.

Die Herstellung der Handreinigungsmittel erfolgt mittels bekannter Vorrichtungen kontinuierlich oder chargenweise. Geeignete Vorrichtungen sind Kessel und Rührwerke, Mischer und Extruder.

Die Verwendung der erfindungsgemäßen Handreinigungsmittel erfolgt, indem zunächst bevorzugt ohne Wasser das Reinigungsmittel auf der Haut verteilt wird, worauf die Reinigung mit Wasser fortgesetzt und unter Abspülen mit Wasser beendet wird.

Die erfindungsgemäßen Handreinigungsmittel zeigen ein gutes Anschäumvermögen, eine gute Reinigungswirkung und verfügen über eine gegenüber herkömmlichen Produkten verbesserte dermatologische Verträglichkeit.

Die Erfindung wird durch folgende Beispiele und Untersuchungen, wie der Hautverträglichkeitsprüfung mit Hilfe des Duhring-Kammer-Testes bzw. des Ellbeugen-Wasch-Testes, der Hautaustrocknung mit Hilfe des Corneometers und der Reinigungskraft mit Hilfe des Handwaschtests und Stabilitätstests beschrieben.

Prüfmethoden:

Hautverträglichkeit mit Hilfe des Duhring-Kammer-Testes nach Frosch, P.J., Kligman, A.M., Contact Dermatites 5, S. 73, 1979 und Kresken, J. Wassilew, S.W., H+G4, S. 334, 1992:

Bei. dieser Methode handelt es sich um ein in vivo-Testmodell zur Überprüfung der Hautverträglichkeit verschiedener Testprodukte im direkten Vergleich. Den Probanden werden die zu testenden Produkte in luftundurchlässigen Aluminiumkammern (Duhring-Kammern) auf die volare Seite der Unterarme an 5 Tagen hintereinander für jeweils 6 Stunden (letzter Tag 5 Stunden) aufjeweils das gleiche Testareal appliziert. Die Befestigung der Duhring-Kammern erfolgt mit Pflasterstreifen. Bei starken dermalen Effekten wird der Test für das jeweilige Testfeld auch vor Erreichen der Gesamtapplikationszeit von 29 Stunden beendet. Beurteilt werden die entstandenen Hautirritationen nach unten angegebener Skala bzw. die Applikationszeit.

R = Rötung (Erythem): 0 = kein Erythem 4 = starkes Erythem

S = Schuppung: 0 = keine Schuppung 4 = starke Schuppung

F = Fissuren: 0 = keine Fissuren 4 = starke Fissuren

Als Beurteilungskriterien ergeben sich die

1. Irritation $\chi$ als Mittelwert aus der Summe der Irritationswerte von R, S und F der n Probanden

2. Applikationszeit $h$ als Mittelwert der tolerierten Applikationszeit in Stunden der n Probanden.

Aus diesen beiden Werten läßt sich die relative Hautverträglichkeit (A-Wert) nach folgender Formel berechnen:

$$\bar{A} = \frac{\bar{h}}{\sqrt{\bar{x}}}$$

Somit ist es möglich, eine Relation zwischen der Applikationszeit und der während dieser Zeit entstandenen Irritation mit einem Wert zu beschreiben. Als Hilfe zur Beurteilung der Hautverträglichkeit der getesteten Produkte läßt sich die folgende Tabelle heranziehen:

A-Wert:

| | |
|---|---|
| > 23 | exzellente Hautverträglichkeit |
| 18 - 23 | sehr gute Hautverträglichkeit |
| 13 - 18 | gute Hautverträglichkeit |
| 8 - 13 | befriedigende Hautverträglichkeit |
| 3 - 8 | ausreichende Hautverträglichkeit |
| < 3 | mangelhafte Hautverträglichkeit |

Prüfung der Hautverträglichkeit nach dem Ellbeugen-Wasch-Test nach P.J. Frosch, Principles of Cosmetics for the Dermatologist, P. Frost, S. Horwitz (Hrsg.), S. 5 - 12, Mosby, St. Louis (1982); M. Puschmann, J. Meyer-Rohn, Ärztl. Kosmetol. 13, 225 (1983).

Der Ellbeugenwaschtest dient u. a. zur Prüfung der Hautverträglichkeit von Grobhandreinigern. Besonders geeignet ist dieser Test für die Beurteilung von reibemittelhaltigen Testprodukten. Aufgrund der mechanisch verursachten Reibung in der Ellenbeuge lassen sich verschiedene reibemittelhaltige Produkte auf ihre Hautreizung prüfen.

An vier aufeinanderfolgenden Tagen werden kontrollierte Waschungen mit den Prüfprodukten an der besonders empfindlichen Haut der Ellenbeuge durchgeführt, bis eine subjektive Bewertung der Hautirritation möglich ist.

Testmaterial:

Zellstoffauflage für sehr dünnflüssige Produkte ( z. B. Pur-Zellin, Firma Hartmann)

Probanden:

Das Testkollektiv besteht aus mindestens 6 hautgesunden Männern und Frauen im Alter von 18 bis 60 Jahren.

Prüfprodukte:

Nicht fließfähige Produkte werden unverdünnt auf die Ellenbeuge gebracht; bei zu großer Trockenheit wird ca. 1 ml Wasser zugefügt.

Flüssige Produkte werden auf Zellstoff getropft und einmassiert.

Testmengen:

Die Prüfprodukte werden in einer Menge von 1 g bzw. 1 ml getestet.

Methode:

Die Prüfprodukte werden randomisiert aufgetragen - der Auftrag erfolgt wie folgt:
50 % der Probanden rechts Produkt A, links Produkt B
50 % der Probanden rechts Produkt B, links Produkt A.

Reibemittelhaltige Grobhandreiniger:

Der Prüfleiter bestimmt je nach zu erwartender Aggressivität der Produkte die Häufigkeit der Waschungen an allen 4 Tagen, pro Tag (ein- bzw. zweimal). Es wird wie folgt geprüft:

Das Testprodukt wird auf die Ellenbeuge gebracht und 2 Minuten mit Zeige- und Mittelfinger verrieben. Anschließend wird das Produkt 2 Minuten auf der Ellbeuge belassen und nachfolgend abgewaschen.

Die Applikation der Prüfprodukte wird vorzeitig abgebrochen, sobald ein Produkt untolerierbare Hautirritationen oder starkes Brennen auf der Haut verursacht.

Bewertung:

Die Bewertung erfolgt visuell durch die/den Versuchsdurchführende/n 24 Stunden nach der letzten tolerierten Waschung im Rechts-Links-Vergleich nach folgenden Bewertungskriterien:

0 = keine Reaktion
1 = schwache fleckige Rötung
2 = mäßige bis mittelstarke Rötung, leicht rauhe Haut, leichte Schuppung
3 = starke mittelflächige Rötung, sehr rauhe Haut, deutliche Schuppung, punktierte Erosionen

Zusätzlich wird das subjektive Empfinden der Probanden nach der folgenden Skala abgefragt:

0 = keine Reaktion
1 = leichtes Spannen der Haut
2 = leichtes Brennen
3 = starkes schmerzendes Brennen

Die Bewertung nach der Punkteskala ermöglicht direkt eine vergleichende Aussage über die Hautverträglichkeit der Produkte. Liegt eine Bewertung zwischen zwei Bewertungspunkten, ist zur weiteren Differenzierung die Beurteilung in 0,5er Schritten möglich.

Prüfung der Hautaustrocknung mit Hilfe des Corneometers:

Die Corneometermessung ist ein non-invasives kapazitives Meßverfahren zur Beurteilung des Feuchtigkeitszustandes der obersten Hautschichten des Stratum corneums. Dieser wird beim hautgesunden Probanden zum größten Teil durch externe Einflüsse bestimmt.

Häufige Hautreinigung führt zu einer Hautentfettung und dadurch zu einer Feuchtigkeitsabnahme in den obersten Hautschichten. Die Abnahme des Feuchtigkeitsgehaltes der Haut stellt eine Verschlechterung des Hautzustandes dar und bedingt ein erhöhtes Ekzemrisiko. Dieser Effekt kann mit dem Corneometer CM 825 der Firma Courage & Khazaka Electronic GmbH, Köln, meßtechnisch erfaßt werden.

Es ist darauf zu achten, daß die Messungen in einem Raum mit konstanten klimatischen Verhältnissen durchgeführt werden. Vor der Messung sind die unbedeckten Hautfelder 15 Minuten zu klimatisieren.

Wird dieses Verfahren zur Unterstützung der Bewertung von Duhring-Kammer-Tests verwendet, so sind vor Testbeginn die Ausgangswerte ($C_o$) zu bestimmen und bei Testende die Endwerte zu ermitteln ($C_n$). Das Feld mit Wasser demin. dient als Kontrollfeld ($C_{kn}$; $C_{ko}$).

Die Änderung der Hautfeuchte C berechnet sich wie folgt:

$$C=(C_n - C_o)-(C_{kn} - C_{ko})$$

Negative Werte bedeuten eine Erhöhung der Hautfeuchte, positive Werte bedeuten eine Verringerung der Hautfeuchtigkeit.

Prüfung der Reinigungskraft mit Hilfe des Handwaschtests:

Das Testmodell des Handwaschtests mit standardisiertem Schmutz oder Lack gibt Auskunft über die Reinigungswirkung der zu prüfenden Produkte. Mit mindestens acht Probanden werden zwei Produkte vergleichend geprüft. Voraussetzung ist, daß alle Probanden eine charakteristische, durch manuelle Arbeit bedingte Hautstruktur der Handinnenflächen besitzen. Morgens und nachmittags wird mit je einem Produkt folgender Test durchgeführt:

- 0,5 g Modellschmutz bzw. 0,5 ml Lack wird auf die Handinnenfläche und auf den Handrücken verteilt und 45 s verrieben.
- 1 ½ min. trocknen lassen
- 1,2 g Testprodukt werden aufgetragen und eingerieben
- 1 ml Wasser wird zugefügt und 30 s gewaschen
- nochmals 1 ml Wasser zufügen und 30 s waschen lassen
- abspülen unter fließendem kalten Wasser
- visuelle Beurteilung der Restverschmutzung (RV) auf dem Handrücken und der Handinnenfläche gemäß Skala s. u.

0 = sauber     5 = kein Reinigungseffekt (Abstufung in 0,5er Schritten möglich)

Die Berechnung des prozentualen Reinigungseffektes erfolgt nach folgender Formel:

$$\text{Reinigungseffekt [\%]} = \frac{10 - (RV_{inner} + RV_{außen})}{10} * 100\ \%$$

$RV_{inner}$ = Mittelwert der Restverschmutzung Handinnenflächen von n Meßreihen (Probanden)
$RV_{außen}$ = Mittelwert der Restverschmutzung Handaußenflächen von n Meßreihen (Probanden)

Da die Bestimmung der Reinigungswirkung infolge der Testmethode eine höhere Schwankungsbreite aufweist, ist eine Absolutabweichung von 5 % zwischen zwei Meßreihen zulässig.

Zusammensetzung des Modellschmutzes:

Motoröl (Castrol) 54,15 %
Vaseline 18,05 %
Adeps lanae 18,05 %
Graphit 3,61 %
Flammruß 5,42 %
Eisenoxid ($Fe_2O_3$) 0,72 %

Ausführungsbeispiele:

Es wurden Handreinigungsmittel gemäß den in Tabelle 1 angegebenen Zusammensetzungen durch Mischen aller Komponenten unter Rühren und bei Raumtemperatur hergestellt. Die Mittel wurden durch die Bestimmung ihrer Hautverträglichkeit, Hautaustrocknung und Reinigungswirkung gegenüber Modellschmutz und Lack charakterisiert. Die ermittelten Ergebnisse sind in der Tabelle 1 zusammengefaßt.

Aus der Tabelle 1 ist zu entnehmen, daß die erfindungsgemäßen Mittel gemäß den Beispielen 1 - 12 in Vergleich zu den lösemittelhaltigen Mitteln der Vergleichsbeispiele 13 - 16 eine bessere Hautverträglichkeit und Hautaustrocknung aufweisen.

Hinsichtlich Reinigungswirkung bei dem verwendeten Modellschmutz zeigen die Rezepturen 1 und 2 eine bessere und die Rezepturen 3 - 12 eine mit den lösemittelhaltigen Rezepturen 13 - 16 vergleichbare Reinigungswirkung.

Hinsichtlich Reinigungswirkung bei dem verwendeten Lack weist die Rezeptur 1 und 2 eine vergleichbare Reinigungswirkung im Vergleich zu DBE-2- oder Di-n-butyladipat-haltigen Rezepturen 13 und 14 aus.

Die erfindungsgemäßen Handreinigungsmittel, wie durch Beispiel 5 ausgewiesen, zeigen auch überraschenderweise eine bessere Reinigungswirkung bei Modellschmutz und Lack als Handreinigungsmittel, die aus DE-C-4038076 insbesondere Beispiel 6 bekannt sind, und eine bessere Hautverträglichkeit mit Hilfe des Ellbeugenwaschtestes ermittelt wurde.

| Rezeptur gemäß Bsp. | 1 | 2 | 3 | 4 | 5 | 6 | 7 | 8 | 9 | 10 | 11 | 12 | 13 | 14 | 15 | 16 | 17* |
|---|---|---|---|---|---|---|---|---|---|---|---|---|---|---|---|---|---|
| Rezeptur gemäß Vergleichsbeispiel | | | | | | | | | | | | | | | | | |
| Rüböl | 30 % | 30 % | 30 % | 20 % | 20 % | 20 % | 10 % | 10 % | 10 % | - | - | - | - | - | - | - | - |
| Traubenkernöl | - | - | - | - | - | - | - | - | - | 20 % | - | - | - | - | - | - | - |
| Sojaöl | - | - | - | - | - | - | - | - | - | - | 20 % | - | - | - | - | - | - |
| Leinöl | - | - | - | - | - | - | - | - | - | - | - | 20 % | - | - | - | - | - |
| Di-n-butyladipat | - | - | - | - | - | - | - | - | - | - | - | - | 20 % | - | - | - | - |
| DBE-2 | - | - | - | - | - | - | - | - | - | - | - | - | - | 20 % | - | - | - |
| Isohexadecan | - | - | - | - | - | - | - | - | - | - | - | - | - | - | 20 % | - | - |
| Terpen | - | - | - | - | - | - | - | - | - | - | - | - | - | - | - | 20 % | - |
| Laureth-6 | 20 % | 20 % | 8 % | 10 % | 10 % | 8 % | 10 % | 8 % | 8 % | 10 % | 10 % | 10 % | 10 % | 10 % | 10 % | 10 % | - |
| Natriumlaurethsulfat | 8 % | 8 % | 1 % | 7 % | 7 % | 1 % | 7 % | 1 % | 1 % | 7 % | 7 % | 7 % | 7 % | 7 % | 7 % | 7 % | - |
| Rizinusölsulfonat | 2 % | 2 % | 1 % | 3 % | 3 % | 1 % | 3 % | 1 % | 1 % | 3 % | 3 % | 3 % | 3 % | 3 % | 3 % | 3 % | - |
| Hautverträglichkeit | 18-23 | 18-23 | 18-23 | 18-23 | 18-23 | 18-23 | 18-23 | 18-23 | 18-23 | 18-23 | 18-23 | 18-23 | 12-17 | 12-17 | <10 | <10 | 18-23 |
| Hautaustrocknung | 0 bis -2 | 0 bis -2 | 0 bis -2 | 0 bis -2 | 0 bis -2 | 0 bis -2 | 0 bis -2 | 0 bis -2 | 0 bis -2 | 0 bis -2 | 0 bis -2 | 0 bis -2 | -4 bis -8 | -12 bis -16 | -4 bis -8 | -12 bis -16 | 0 bis -2 |
| Reinigungswirkung | | | | | | | | | | | | | | | | | |
| Modellschmutz | 95-98 % | 95-98 % | 90-95 % | 85-90 % | 90-95 % | 90-95 % | 90-95 % | 90-95 % | 90-95 % | 90-95 % | 90-95 % | 90-95 % | 90-95 % | 90-95 % | 90-95 % | 90-95 % | 80-85 % |
| Lack | 90-95 % | 90-95 % | 80-85 % | 80-85 % | 85-90 % | 80-85 % | 85-90 % | 83-88 % | 80-85 % | 80-85 % | 80-85 % | 80-85 % | 90-95 % | 90-95 % | 80-85 % | 80-85 % | 70-75 % |
| Hautverträglichkeit (Ellbeugenwaschtest) — Score | 0,4-0,6 | 0,8-1,0 | | 0,5-0,7 | 1,0-1,2 | | | | | | | | | | | | 1,2-1,4 |
| subjectiv | 0,2-0,4 | 0,4-0,6 | | 0,2-0,4 | 0,4-0,6 | | | | | | | | | | | | 0,5-0,7 |

Angaben der Zusammensetzung der Rezepturen in Gewichts-%; alle Zusammensetzungen enthalten 5 Gew.% Verdickungsmittel (organophiler Bentonit, Carboxymethylcellulose, NaCl) und Wasser; sowie Zusätze von 1 Gew.% (insgesamt) von Zitronensäure, Konservierungsmittel, Vitamin E-Acetat. Die Zusammensetzungen 2, 3, 5 bis 7 und 9 bis 12 und die Vergleichsbeispiele 13 bis 17 enthalten 13 Gew.% Walnußschalenmehl.

* Vergleichsbeispiel 17 entspricht Beispiel 6 aus DE-C-4038076

**Patentansprüche**

1.  Wasserhaltiges, flüssiges, pasten- oder cremeförmiges Handreinigungsmittel, das kein organisches Lösungsmittel enthält und **gekennzeichnet ist durch** einen Gehalt an

    a) 10 bis 30 Gew.%, bezogen auf die Zusammensetzung des Handreinigungsmittels, eines oder mehrerer pflanzlicher Öle aus der Gruppe Triglyceride gesättigter und/oder ungesättigter Fettsäuren,

    b) 10 bis 30 Gew.%, bezogen auf die Zusammensetzung des Handreinigungsmittels, einer Tensidkomposition, die

      α) wenigstens ein Fettalkoholethoxylat
      β) wenigstens ein Fettalkoholethersulfat
      γ) wenigstens ein Salz einer sulfierten, vorzugsweise sulfonierten Fettsäure enthält,

    c) 10 bis 65 Gew.%, bezogen auf die Zusammensetzung des Handreinigungsmittels, Wasser,

    d) gegebenenfalls 1 bis 30 Gew.%, bezogen auf die Zusammensetzung des Handreinigungsmittels, eines oder mehrerer Abrasiva,

    e) gegebenenfalls ein oder mehrere viskositätsbildende Mittel,

    f) gegebenenfalls weitere kosmetische Hilfs-, Zusatz- und/oder Wirkstoffe,

    wobei die Summe der Komponenten a) bis f) immer 100 Gew.% ergeben muß und wobei der Gehalt der Komponente a) größer oder gleich dem Gehalt an Fettalkoholethoxylaten α) und der Gehalt an Fettalkoholethoxylaten α) größer oder gleich dem Gehalt der beiden Komponenten β) und γ) in der Zusammensetzung des Handreinigungsmittels ist.

2.  Handreinigungsmittel nach Anspruch 1, **dadurch gekennzeichnet, daß** als Triglyceride Triglyceride mit einem höheren Anteil an ungesättigten Fettsäuren, vorzugsweise Rüböl, Sojaöl und/oder Leimöl, enthalten sind.

3.  Handreinigungsmittel nach Anspruch 1 oder 2, **dadurch gekennzeichnet, daß** die Tensidkomposition b) als Fettalkoholethoxylate, solche der allgemeinen Formel

$$R\text{-}O(CH_2\text{-}CH_2\text{-}O)_nH$$

    mit
    R = einem $C_8$-$C_{18}$, vorzugsweise $C_{10}$-$C_{16}$, besonders bevorzugt $C_{11}$-$C_{14}$ gesättigten oder ungesättigten, verzweigteri oder unverzweigten Alkylrest und n = einer ganzen Zahl von 1 bis 8, vorzugsweise 3 bis 6, besonders bevorzugt 5 bis 7,
    als Fettalkoholethersulfate solche der allgemeinen Formel

$$R\text{-}O\text{-}(CH_2\text{-}CH_2\text{-}O)_nSO_3X$$

    mit R = einem $C_{10}$-$C_{16}$, vorzugsweise $C_{11}$-$C_{14}$ gesättigten oder ungesättigten, verzweigten oder unverzweigten Alkylrest, n = 1 bis 6, vorzugsweise 1 bis 4, und X = $Na^+$, $NH_4^+$ oder $Mg^{2+}$, vorzugsweise $Na^+$,
    und als Salze sulfierter, vorzugsweise sulfonierter Fettsäuren, Alkali-, Erdalkali- oder Ammonium-Salze von $C_8$-$C_{30}$, bevorzugt $C_{10}$-$C_{22}$-Fettsäuren, vorzugsweise Natrium- oder Ammonium-Rizinusölsulfat enthält.

4.  Handreinigungsmittel nach Anspruch 3, **dadurch gekennzeichnet, daß** als Tensidkomposition b) Laureth-6, Natriumlaurylethersulfat und Natrium- oder Ammonium-Rizinusölsulfonat eingesetzt wird.

5.  Handreinigungsmittel nach einem oder mehreren Ansprüchen 1 bis 4, **dadurch gekennzeichnet, daß** die Tensidkomposition b) aus

5 bis 28 Gew.%, vorzugsweise 7 bis 25 Gew.% der Komponente α),
1,9 bis 15 Gew.%, vorzugsweise 3 bis 30 Gew.% der Komponente β) und
0,1 bis 10 Gew.%, vorzugsweise 0,5 bis 6 Gew.% der Komponente γ) besteht,

wobei die Summe der Komponenten α) bis γ) 10 bis 30 Gew.% ergeben muß.

**6.** Handreinigungsmittel nach einem oder mehreren Ansprüchen 1 bis 5, **dadurch gekennzeichnet, daß** als Abrasiva Kunststoffreibemittel auf Basis von Polyethylen oder Polyurethan, Reibemittel auf Basis von natürlichen Kernund/ oder Schalenmehlen, bevorzugt gebleichten natürlichen Schalenund/oder Kernmehle, und/oder Perlen aus Wachsen eingesetzt werden.

**7.** Handreinigungsmittel nach Anspruch 6, **dadurch gekennzeichnet, daß** mit $H_2O_2$ gebleichtes Walnußschalenmehl oder Jojobawachsperlen eingesetzt werden.

**8.** Handreinigungsmittel nach einem oder mehreren Ansprüchen 1 bis 7, **dadurch gekennzeichnet, daß** als viskositätsbildende Mittel organophile und/oder hydrophile Schichtsilikate, bevorzugt Bentonite, Polysaccharide, bevorzugt Cellulose, Guarmehl und/oder Xanthane, modifizierte Polysaccharide, vorzugsweise Celluloseether, Carboxymethylcellulose und/oder Hydroxyethylcellulose und/oder anorganische Elektrolyte, vorzugsweise Natriumchlorid und/oder Magnesiumsulfat, eingesetzt werden.

**9.** Handreinigungsmittel nach einem oder mehreren Ansprüchen 1 bis 8, **dadurch gekennzeichnet, daß** als kosmetische Hilfs-, Zusatz- und/oder Wirkstoffe, pH-Regulatoren, Duftstoffe, Konservierungsmittel, vorzugsweise organische Säuren, Antioxidantien, vorzugsweise Vitamin-E-Acetat, eingesetzt werden.

**10.** Handreinigungsmittel nach einem oder mehreren der Ansprüche 1 bis 9, **dadurch gekennzeichnet, daß** als Komponente a) Rüböl, als Komponente b) eine Mischung aus Laureth-6, Natriumlaurylethersulfat und Rizinusölsulfonat, als Komponente d) gebleichtes Walnußschalenmehl, als Komponente e) Bentonit, Carboxymethylcellulose und Natriumchlorid und als Komponente f) Zitronensäure, Konservierungsmittel, Vitamin-E-Acetat und Parfüm enthalten sind.

**Claims**

**1.** A water-containing, fluid hand cleaning agent in paste or cream form, containing no organic solvent and **characterised in that** it comprises:

a) 10 to 30% by weight, with reference to the composition of the hand cleaning agent, of one or more vegetable oils of the group comprising saturated and/or unsaturated triglyceride fatty acids,

b) 10 to 30% by weight, with reference to the composition of the hand cleaning agent, of a surfactant composition including:

α) at least one fatty alcohol ethoxylate

β) at least one fatty alcohol ether sulphate

γ) at least one salt of a sulphated, preferably sulphonated, fatty acid

c) 10 to 65% by weight, with reference to the composition of the hand cleaning agent, of water,

d) optionally 1 to 30% by weight, with reference to the composition of the hand cleaning agent, of one or more abrasives,

e) optionally one or more viscosity enhancing substances,

f) optionally further cosmetic aid-, additive- and/or active ingredients,

whereby the sum of the components a) to f) must always total 100% and wherein the content in the composition of the hand cleaning agent of components a) is greater than or equal to the content of fatty alcohol ethoxylates $\alpha$), and the content of fatty alcohol ethoxylates $\alpha$) is greater than or equal to the content of both components $\beta$) and $\gamma$).

2. A hand cleaning agent as claimed in claim 1, **characterised in that** the triglycerides comprise triglycerides having a higher proportion of unsaturated fatty acids, preferably rape seed oil, soya oil and/or linseed oil.

3. A hand cleaning agent as claimed in claim or 2, **characterised in that** the surfactant composition b) includes:

   as the fatty alcohol ethoxylate those having the general formula:

   $$R\text{-}O\text{-}(CH_2\text{-}CH_2\text{-}O)_nH$$

   where R is a $C_8$-$C_{18}$, preferably a $C_{10}$-$C_{16}$, more preferably a $C_{11}$-$C_{14}$, saturated or unsaturated, branched or non-branched alkyl residue and n is an integer from 1 to 8, preferably 3 to 6, more preferably 5 to 7;

   as the fatty alcohol ether sulphate those having the general formula:

   $$R\text{-}O\text{-}(CH_2\text{-}CH_2\text{-}O)_nSO_3X$$

   where R is a $C_{10}$-$C_{16}$, preferably a $C_{11}$-$C_{14}$, saturated or unsaturated, branched or non-branched alkyl residue, n is 1 to 6, preferably 1 to 4, and X is $Na^+$, $NH_4^+$ or $Mg^{2+}$, preferably $Na^+$;

   and as the salts sulphated, preferably sulphonated, fatty acids, alkaline-, alkaline earth- or ammonium-salts of $C_8$-$C_{30}$, preferably $C_{10}$-$C_{22}$, fatty acids, preferably sodium- or ammonium-castor oil sulphonate.

4. A hand cleaning agent as claimed in claim 3, **characterised in that** the surfactant composition b) further includes laureth-6, sodium lauryl ether sulphate and sodium- or ammonium-castor oil sulphonate.

5. A hand cleaning agent as claimed in one or more of claims 1 to 4, **characterised in that** the surfactant composition b) comprises:

   5 to 28% by weight, preferably 7 to 25% by weight, of component $\alpha$),

   1.9 to 15% by weight, preferably 3 to 30% by weight, of component $\beta$), and

   0.1 to 10% by weight, preferably 0.5 to 6% by weight, of component $\gamma$),

   whereby the sum of components $\alpha$) to $\gamma$) must total 10 to 30% by weight.

6. A hand cleaning agent as claimed in one or more of claims 1 to 5, **characterised in that** the abrasives are plastics abrasive agents based on polyethylene or polyurethane, abrasive agents based on natural kernel and/or shell flours, preferably bleached natural kernel and/or shell flours, and/or beads made of wax.

7. A hand cleaning agent as claimed in claim 6, **characterised in that** there is incorporated walnut shell flour bleached with $H_2O_2$ or jojoba wax beads.

8. A hand cleaning agent as claimed in one or more of claims 1 to 7, **characterised in that** the viscosity enhancing substances comprise organophilic and/or hydrophilic layer silicates, preferably bentonites, polysaccharides, preferably cellulose, guar flour and/or xanthans, modified polysaccharides, preferably cellulose ether, carboxymethyl cellulose and/or hydroxyethyl cellulose and/or inorganic electrolytes, preferably sodium chloride and/or magnesium sulphate.

9. A hand cleaning agent as claimed in one or more of claims 1 to 8, **characterised in that** the cosmetic aid-, additive- and/or active ingredients comprise pH regulators, perfumes, preservatives, preferably organic acids, antioxidants, preferably vitamin E acetate.

**10.** A hand cleaning agent as claimed in one or more of claims 1 to 9, **characterised in that** component a) comprises rape seed oil, component b) comprises a mixture of laureth-6, sodium laureth ether sulphate and castor oil sulphonate, component d) comprises bleached walnut shell flour, component e) comprises bentonite, carboxymethyl cellulose and sodium chloride and component f) comprises citric acid, preservative, vitamin E acetate and perfume.

**Revendications**

**1.** Détergent liquide destiné aux mains contenant de l'eau, sous la forme de pâte ou de crème , ne comportant aucun solvant organique et **caractérisé par** une teneur de

a) 10 à 30 % en poids, par rapport à la composition du détergent destiné aux mains, de une ou de plusieurs huiles végétales du groupe triglycéride d'acides gras saturés et / ou non saturés,
b) 10 à 30 % en poids, par rapport à la composition du détergent destiné aux mains, d'une composition d'agent tensioactif qui comporte :

$\alpha$) au moins un éthoxylate d'alcool gras
$\beta$) au moins un sulfate d'éther d'alcool gras
$\gamma$) au moins un sel d'acide gras sulfuré, de préférence sulfoné

c) 10 à 65 % en poids d'eau, par rapport à la composition du détergent destiné aux mains,
d) éventuellement, 1 à 30 % en poids, d'un ou plusieurs abrasifs , par rapport à la composition du détergent destiné aux mains,
e) éventuellement un ou plusieurs agents fournissant de la viscosité
f) éventuellement d'autres adjuvants, additifs et / ou agents actifs,

la somme des composants a) à f) devant être 100% en poids et la teneur des composants a) est supérieure ou égale à la teneur en éthoxylate d'alcool gras $\alpha$) et la teneur en éthoxylate d'alcool gras $\alpha$) est supérieure ou égale à la teneur des deux composants $\beta$) et $\gamma$) dans la composition du détergent destiné aux mains.

**2.** Détergent destiné aux mains selon la revendication 1, **caractérisé en ce qu'**il contient du triglyceride avec une teneur supérieur en acide gras non saturé de préférence de l'huile de navette , de l'huile de soja et / ou de l'huile de lin .

**3.** Détergent destiné aux mains selon une des revendications 1 ou 2, **caractérisé en ce que** la composition de l'agent tensioactif b) comporte des éthoxylates d'alcool gras comme ceux de la formule générale suivante :

$$R-O(CH_2-CH_2-O)_nH$$

avec
R = un reste alkylique C8 - C16, de préférence $C_{10}$ - $C_{16}$, particulièrement préféré $C_{11}$ - $C_{14}$, saturé ou non saturé , ramifié ou non ramifié, et n = un nombre entier 1 à 8, de préférence 3 à 6, particulièrement préféré de 5 à 7, et sont contenus en tant que sulfate d'éther d'alcool gras ayant la formule générale suivante :

$$R-O-(CH_2-CH_2-O)_nSO_3X$$

avec
R = un reste alkylique C10 - C16, de préférence $C_{11}$ - $C_{14}$, saturé ou non saturé, ramifié ou non ramifié, n = 1 à 6, de préférence 1 à 4, et X = $Na^+$, $NH_4^+$, ou $Mg_2^+$, de préférence $Na^+$, et sont contenus en tant que sels d'acides gras sulfurés ou de préférence sulfonés des sels alcalins, sels alcalino-terreux et d'ammonium d'acides gras $C_8$ - $C_{30}$, de préférence $C_{10}$ - $C_{22}$, de préférences du sulfate d'huile de ricin de natrium ou d'ammonium.

**4.** Détergent destiné aux mains selon la revendication 3, **caractérisé en ce qu'**on utilise comme composition d'agent tensioactif b) du laureth - 6, du sulfate d'éther laurique de natrium et du sulfonate d'huile de ricin de natrium ou d'ammonium.

5. Détergent destiné aux mains selon une ou plusieurs des revendications 1 à 4, **caractérisé en ce que** la composition de l'agent tensioactif b) est composé de :

5 à 28 % en poids , de préférence 7 à 25% en poids du composant $\alpha$),
1,9 à 15 % en poids, de préférence 3 à 30% en poids du composant $\beta$) et
0,1 à 10 % en poids , de préférence 0,5 à 6% en poids du composant $\gamma$), où la somme des composants $\alpha$) à $\gamma$) doit être 10 à 30% en poids.

6. Détergent destiné aux mains selon une ou plusieurs des revendications 1 à 5, **caractérisé en ce qu'**on utilise comme abrasifs des agents abrasifs en matière plastique sur la base de polyéthylène ou de polyuréthane, des agents pour frotter sur la base de farines naturelles de noyaux et / ou de coquille, de préférence des farines naturelles blanchies de noyaux ou de coquilles , et / ou des perles de résines.

7. Détergent destiné aux mains selon la revendication 6, **caractérisé en ce qu'**on utilise de coquille de noix blanchie au moyen de $H_2O_2$ ou des perles de résine de jojoba.

8. Détergent destiné aux mains selon une ou plusieurs des revendications 1 à 7 , **caractérisé en ce qu'**on utilise comme agents fournissant de la viscosité des silicates stratifiés organophiles et / ou hydrophiles, de préférence des bentonites, des polysaccharides de préférence de la cellulose, de la farine de guar et / ou xanthane, des polysaccharides modifiés, de préférence de l'éther de cellulose, la cellulose carboxyméthylique et / ou hydroxyméthylique et / ou des électrolytes anorganiques , de préférence du chlorure de natrium et / ou du sulfate de magnésium.

9. Détergent destiné aux mains selon une ou plusieurs des revendications 1 à 8, **caractérisé en ce qu'**on utilise des adjuvants, additifs et / ou des agents actifs , des régulateurs de pH, des parfums, des conservateurs, de préférence des acides organiques, des antioxydants, de préférence d'acétate de vitamine E.

10. Détergent destiné aux mains selon une ou plusieurs des revendications 1 à 9, **caractérisé en ce qu'**on utilise comme composant a) de l'huile de navette, comme composant b) un mélange de laureth - 6, sulfate d'éther laurique de natrium et de sulfonate d'huile de ricin de natrium et d'ammonium, comme composant d) de la farine de coquille de noix blanchie, comme composant e) du bentonite, de la cellulose carboxyméthylique et du chlorure de natrium et comme composant f) de l'acide citrique, des conservateurs, de l'acétate de vitamine E et du parfum.